# EUROPEAN PATENT APPLICATION

(11) **EP 3 647 405 A1**
(43) Date of publication of application: **06.05.2020**
(21) Application number: 18306441.9
(22) Date of filing: 02.11.2018
(51) Int. Cl.: C12M 1/00, B01L 3/00, C12M 3/06

(54) **CELL CULTURE DEVICE AND METHOD OF USING THE SAME**

(71) Applicant: Microfluidx, London, Greater London W9 1AU (GB)
(72) Inventor: CEJAS, Cesare, 92160 ANTONY (FR); ALLAF-AKBARI, Elyar, Toronto Ontario M3A2A7, (CA); ESPINET, Antoine, London W9 1 AU (GB)
(74) Representative: Regimbeau

(57) **Abstract**

The present invention relates to a cell culture device comprising at least a fluidic channel having at least a cell medium inlet and a cell medium outlet, said fluidic channel comprising a lower wall extending between the cell medium inlet and the cell medium outlet, the cell culture device further comprising at least one recess configured to receive a plurality of cells, said recess being formed by the lower wall and defining a bottom surface, and at least a cell trap, said cell trap being configured to capture a cell advected in the fluidic channel and then to make the cell sediment to the bottom surface of the recess.

## Description

### TECHNICAL FIELD

The present invention relates to a cell culture device and to a method of cell culture, using such a device, the method comprising seeding, washing, sampling, differentiation, transduction, expansion and/or harvesting of cells in the device. The device according to the invention aims at performing the principal cell culture steps in the same device without transporting the cultured cells in different chambers of the device or to other external chambers outside of the device, in order to produce cells at a high throughput for cell therapy.

### BACKGROUND OF THE INVENTION

Cell therapy is a process in which functional cells are administered into a patient. The production of cells represents significant challenges for regulatory authorities, manufacturers, health care providers and patients involved in their application, since it requires the extraction of cells from a patient, the culture and/or reprogramming of the cells, and the introduction of the cells into the patient.

Typically, the cell culture can comprise at least a seeding step in a bioreactor, a treatment step (for example a step of transduction of the cells), a proliferation or expansion step, and a harvesting step. Samples must be taken at regular intervals for quality control purposes. In conventional methods, the cells are typically cultured in cell culture dishes such as Petri dish, flasks, bottles or bags. Generally, multiple dishes have to be used during the expansion phase in order to keep cell density viable for the cells (usually lower than confluence density). Cell culture dishes are not adapted to a finely tuned control of the cell environment, such as the concentration of nutrients, metabolites and other agents driving differentiation and expansion. The efficiency and the throughput of the cell culture is therefore limited when using cell culture dishes. Moreover, handling of the cells between different dishes and handling of the cell medium in and out of the dishes increase the risk of a cell culture contamination and the cost of the cell culture.

Therefore, bioreactors fabricated with microfluidics technology have been developed to increase the cell cultures throughput and/or to increase the control of each step and/or to study at least one of the cell culture steps.

EP 3029135 A1 describes a microfluidic cell culture system, comprising a plurality of culture units, each culture unit comprising at least a culture area, a cell loading inlet and a medium channel. The culture unit is separated of the medium channel by a structure providing a fluidic connection between the medium channel and the culture unit, and protecting the cells from being advected outside from the cell culture area. Therefore, the cells can be cultured in a compact space and the medium in contact with the cells can be controlled without moving the cells in different bioreactors. Moreover, aggregate of cultured cells can be maintained in the cell culture area. However, the system is not adapted for harvesting the cultured cells.

WO 2007024701 A3 describes a cell culture microfluidic device adapted for cell culture monitoring. The device comprises a microfluidic channel, said channel comprising a plurality of weir-traps. Each weir-trap is adapted to capture a cell. The adherence and the divisions of each cell can be monitored under dynamic control of the medium in the microfluidic channel. However, the device is not adapted for high throughput cell production: the growth of the cell population in each weir-trap is limited to two cells. Moreover, harvesting of the cells is not controllable.

Rousset *et al.* (Rousset, N., Monet, F., & Gervais, T., 2017, Simulation-assisted design of microfluidic sample traps for optimal trapping and culture of non-adherent single cells, tissues, and spheroids. Scientific reports, 7(1), 245) describe a device for non-adherent cell culture. The device comprises a microfluidic channel, said channel comprising a plurality of recesses. Each recess is adapted to trap a single cell. When trapped, a cell is protected by the recess from lifting in the mainstream, while nutriments or drugs can be provided to the cell from the mainstream by diffusion. Therefore, no structure is needed to separate a cell in the recess from the rest of the microfluidic channel, simplifying the fabrication of the cell culture device. However, the device is not adapted for the expansion or the production of cells, since each trap is adapted to trap a single cell.

### SUMMARY OF THE INVENTION

A cell culture device has been developed to respond at least partially to the above-mentioned issues of the prior art.

The cell culture device has at least one surface portion defining a planar surface, the surface portion being configured for lying on a horizontal support, comprising at least a fluidic channel having at least a cell medium inlet and a cell medium outlet, said fluidic channel comprising a lower wall extending between the cell medium inlet and the cell medium outlet, the cell culture device being characterized in that it further comprises:
- at least one recess configured to receive a plurality of cells, said recess being formed by the lower wall and defining a bottom surface and
- at least a cell trap, said cell trap being configured to capture a cell advected in the fluidic channel and then to make the cell sediment to the bottom surface of the recess.

In further optional aspects of the invention:
- the lower wall further comprises an upper surface extending aside of the recess and the cell traps comprises an obstacle extending from the bottom surface and out of the recess beyond the upper surface of the lower wall,
- the fluidic channel further comprises an upper wall, and the cell trap comprises an obstacle extending at least from the upper wall to the recess,
- the obstacle has a main direction of extension defining a sedimentation axis, which is parallel to a sedimentation direction of the cell within said obstacle, and wherein said sedimentation axis is perpendicular to the surface portion,
- each obstacle has an aperture, notably a traversing aperture and preferentially a traversing slit, a width of the aperture being comprised between 3 µm and 30 µm, and preferentially between 5 µm and 20 µm,
- at least a portion of the fluidic channel is configured for receiving a liquid flow according to a main flow direction extending between the cell medium inlet and the cell medium outlet, and the fluidic channel comprises an array of cell traps, said array of cell traps extending along a direction which is perpendicular to the main flow direction in a plane parallel to the planar surface,
- the fluidic channel further comprises lateral sides extending between the cell medium inlet and the cell medium outlet, and wherein at least a recess extends over the entire width of the fluidic channel from one lateral side to the other,
- the lower wall further comprises an upper surface extending aside of the recess and the fluidic channel further comprising an upper wall, a first height of the fluidic channel being defined as a shorter distance between the upper surface of the lower wall and the upper wall, a second height of the recess is defined as a shorter distance between the bottom surface of the lower wall and the upper surface of the lower wall, the second height being comprised between 0.15 and 0.85 times the first height and preferentially between 0.20 and 0.70 times the first height,
- the lower wall further comprises an upper surface extending aside of the recess, a second height of the fluidic channel being defined as a shorter distance between the lower surface of the lower wall and the upper surface of the lower wall, and the second height being comprised between 10 µm and 500 µm, notably between 15 µm and 300 µm and preferably between 15 µm and 200 µm,
- a minimum distance between two cell traps is comprised between 10 µm and 300 µm, notably between 20 µm and 150 µm and preferentially between 25 m and 100 µm,
- the cell culture device comprises at least a harvesting inlet and a harvesting outlet, the harvesting inlet and the harvesting outlet being different from the cell medium inlet and the cell medium outlet, the at least one recess being fluidically directly connected to a harvesting inlet and to a harvesting outlet,
- the cell culture device comprises an inlet channel and an outlet channel, the cell culture device comprising a plurality of fluidic channels each having at least a respective cell medium inlet and cell medium outlet, the inlet channel fluidically connecting the cell medium inlet of each fluidic channel and the outlet channel fluidically connecting the cell medium outlet of each fluidic channel,

Another aspect of the present invention is a method of cell culture, comprising setting the cell culture device so that the surface portion lies on a horizontal support, and comprising a seeding step a) of injecting a medium comprising cells, said medium being injected in the cell medium inlet of the at least one fluidic channel of the cell culture device, until at least more than 10% of the cell traps have captured at least one cell, and preferentially more than the majority of the cell traps have captured at least one cell.

In further optional aspects of the invention:
- the method comprises, further to seeding step a), an expansion step b) of injecting a medium comprising an active culture agent in the cell medium inlet, wherein the medium is injected at a first predefined flow rate, the method comprising an optional harvesting step e), further to seeding step a), of injecting a medium in the cell medium inlet at a predefined second flow rate and recouping cells from the cell medium outlet, the second flow rate being strictly higher than the first flow rate, the method further comprising an optional sampling step f), further to step a), of injecting a medium in the cell medium inlet at a predefined fifth flow rate and recouping a sample of cells from the cell medium outlet, the fifth flow rate being strictly higher than the first flow rate, the injection time and the fifth sampling flow rate being calculated previously to seeding step a) so that less than one third of the cells in the recesses are recouped,
- the method comprises further to seeding step a), a washing step c) of injecting a washing medium in the cell medium inlet, wherein the medium is optionally injected at a third predefined flow rate, the method further comprising an optional harvesting step e), further to seeding step a), of injecting a medium in the cell medium inlet at a predefined second flow rate and recouping cells from the cell medium outlet, the second flow rate being strictly higher than the third flow rate, the method further comprising an optional sampling step f), further to step a), of injecting a medium in the cell medium inlet at a predefined fifth flow rate and recouping a sample of cells from the cell medium outlet, the fifth flow rate being strictly higher than the third flow rate, the injection time and the fifth sampling flow rate being calculated previously to seeding step a) so that less than one third of the cells in the recesses are recouped,
- the method comprises, further to seeding step a), a differentiation step d) of injecting a medium comprising at least a differentiation factor in the cell medium inlet, wherein the medium is optionally injected at a fourth predefined flow rate, the method further comprising an optional harvesting step e), further to seeding step a), of injecting a medium in the cell medium inlet at a predefined second flow rate and recouping cells from the cell medium outlet, the second flow rate being strictly higher than the fourth flow rate, the method further comprising an optional sampling step f), further to step a), of injecting a medium in the cell medium inlet at a predefined fifth flow rate and recouping a sample of cells from the cell medium outlet, the fifth flow rate being strictly higher than the fourth flow rate, the injection time and the fifth sampling flow rate being calculated previously to seeding step a) so that less than one third of the cells in the recesses are recouped,
- the method comprises, further to seeding step a) a harvesting step e) of injecting a medium in the cell medium inlet(s) preferentially and recouping at least 80% of cells from the cell medium outlet(s).
- the cell culture device comprises at least a harvesting inlet and a harvesting outlet, the harvesting inlet and the harvesting outlet being different from the cell medium inlet and the cell medium outlet, at least one recess being fluidically directly connected to a harvesting inlet and to a harvesting outlet, the method further comprising, further to step a), a harvesting step e) of injecting a medium in the harvesting inlet(s) and recouping cells from the harvesting outlet(s),
- the method further comprises, further to seeding step a), a transduction step of injecting a medium comprising a transduction factor, said medium being injected in the cell medium inlet at a predefined flow rate comprised between 0.5 and 1.5 times the fourth predefined flow rate.

### DEFINITIONS

The term *"length"* of a channel will be used herein to designate the size of a channel according to the main flow direction of a fluid through the channel.

The term *"height"* of a channel will be used herein to designate the minimum size of a channel in the first direction, said first direction being transverse to the main flow direction.

The term *"width"* of a channel will be used herein to designate the maximum size of a channel in the second direction perpendicular to the first direction and perpendicular to the main flow direction.

The terms *"microchannel"* or *"microfluidic channel"* will be used herein to designate a channel comprising at least one inlet and at least one outlet, the height of which is comprised between 100 nm and 1 mm. The term *"fluidic channel"* also comprises a range of dimensions including but not limited to nano-, micro-, milli, - centi- dimensions.

### BRIEF DESCRIPTION OF THE DRAWINGS

The invention will be described by way of example, with reference to the accompanying drawings in which:
- figure 1 illustrates a cell culture device,
- figure 2 illustrates diagrammatically a portion of a fluidic channel comprising a recess and a cell trap,
- figure 3 is a top view of a cell trap,
- figure 4 illustrates diagrammatically a section of a fluidic channel along the main flow direction,
- figure 5 is a top view of a fluidic channel,
- figure 6 is a top view of a fluidic channel comprising harvesting inlets and harvesting outlets,
- figure 7 illustrates a method of cell culture according to a possible embodiment of the invention.

### DETAILED DESCRIPTION OF PREFERRED ASPECTS OF THE INVENTION

### General architecture of the cell culture device 1

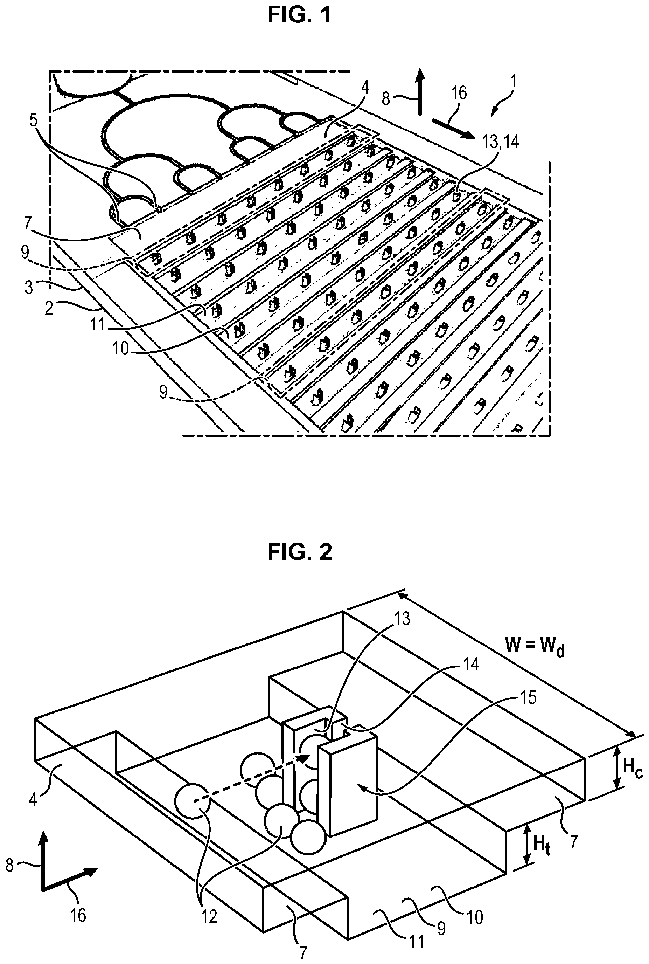

Referring to figure 1, the cell culture device 1 has a surface portion 2 defining a planar surface 3, configured for lying on a horizontal support. The surface portion 2 is behind the illustrated part of the cell culture device 1 in figure 1. Depending on the fabrication method of the cell culture device 1, the surface portion 2 can be, for example, the surface of a glass slide or a planar surface in reticulated polymer, for example of PDMS.

The cell culture device 1 comprises at least a fluidic channel 4. The fluidic channel 4 is preferentially a microfluidic channel. The fluidic channel 4 has at least a cell medium inlet 5. According to the possible embodiment of the invention illustrated in figure 1, the fluidic channel 4 has eight cell medium inlets 5, uniformly distributed at one end of the fluidic channel 4. Therefore, it is possible to inject a homogeneous concentration of cells 12 in the fluidic channel 4 and/or to inject a liquid medium at the same velocity across a section of the fluidic channel 4.

The fluidic channel 4 comprises at least a cell medium outlet 6 (not illustrated in figure 1). The cell medium outlet(s) is (are) at another end of the fluidic channel 4.

A first direction 8 is defined perpendicular to the planar surface 3, and opposite to a direction going towards the planar surface 3 from the fluidic channel 4. In use, the cell culture device 1 lies on a horizontal support and the first direction 8 corresponds to a direction opposite to the direction of gravity.

The fluidic channel 4 comprises a lower wall 7 extending between the cell medium inlet and the cell medium outlet.

Referring to figure 1 and to figure 2, the fluidic channel 4 comprises at least one, and preferably a plurality of recesses 10, each recess 10 being configured to receive a plurality of cells 12. The recess 10 is formed by the lower wall 7. According the possible embodiment of the invention illustrated in figure 1 and in figure 2, the recess 10 has a rectangular cuboid shape, and is fabricated for example, but not limited to, by a two-step photolithography microfabrication technique. The recess 10 defines at least a bottom surface 11. In the case of a recess 10 having a rounded shape, the bottom surface 11 can define the entire recess 10. The bottom surface 11 is the lowest surface of the recess relative to the first direction 8.

The cell culture device 1 comprises at least one, and preferably a plurality of cell traps 13. Each cell trap 13 is adapted to capture a cell 12 advected by a liquid medium flow in the fluidic channel 4, and then to make the captured cell 12 sediment onto the bottom surface 11 of the recess 10. A cell culture zone 9 comprises a recess 10 and at least a cell trap 13.

### Cell traps 13

Referring to figure 2, the lower wall 7 comprises an upper surface 23, corresponding to the surface extending aside of the recess 10. The cell trap 13 is preferentially an obstacle 14. The obstacle 14 can be arranged on the boundary between the recess 10 and the rest of the fluidic channel 4. Preferentially, the cell trap 13 comprises an obstacle 14, extending from the bottom surface 11, out of the recess 10, beyond the upper surface 23 of the lower wall 7. Preferentially, the cell trap 13 comprises an obstacle 14 extending at least from the upper wall 20 to the recess 10. The obstacle 14 has preferentially a main direction of extension defining a sedimentation axis, which is parallel to the sedimentation direction of the cell 12 within said obstacle 14, said sedimentation axis being perpendicular to the surface portion 2. The sedimentation axis can correspond to the first direction 8. The obstacle 14 preferentially extends all along a section of the fluidic channel 4, relative to the axis, from the bottom surface 11 of the recess 10 to the upper wall 20 of the fluidic channel 4. Therefore, the capture efficiency of a cell 12 by the obstacle 14 is increased.

When a cell 12 is advected by the liquid flow, flowing for example in the main flow direction 16, the cell 12 can meet the obstacle 14 and be stopped from being advected in the main flow direction 16. However, the obstacle 14 is configured for letting the cell 12 sediment in the recess 10 to the bottom surface 11. The capture and sedimentation of a cell 12 are illustrated by the dotted arrows in figure 2. Therefore, the cells 12 can be trapped efficiently without limiting the velocity of the injected medium comprising cells 12 in order for the cells 12 to sediment in the recess 10. By the combination of the cell traps 13 and the recesses 10, it is possible to control a liquid medium comprising cells 12 in the fluidic channel 4 at a speed at which sedimentation is typically neglected, while using sedimentation to seed cells 12 in the recesses 10. The seeding of the cells 12 is simpler and more efficient than in the prior art.

The obstacle 14 can be a weir-trap. Therefore, the cell medium streamlines directed at the obstacle 14 do not all circumvent the obstacle 14. Some of the streamlines pass by the obstacle 14. Therefore, if a cell 12 is in the streamline passing through the obstacle 14, the cell 12 can be captured by the obstacle 14. After a cell 12 is captured, the cell 12 partially occludes the open region of the weir-trap, and the fraction of streamlines through the barred trap decreases, leading to the self-sealing of the traps. It is therefore possible to choose the dimension of the weir-trap in order to control the number of captured cell(s) 12 by weir-trap.

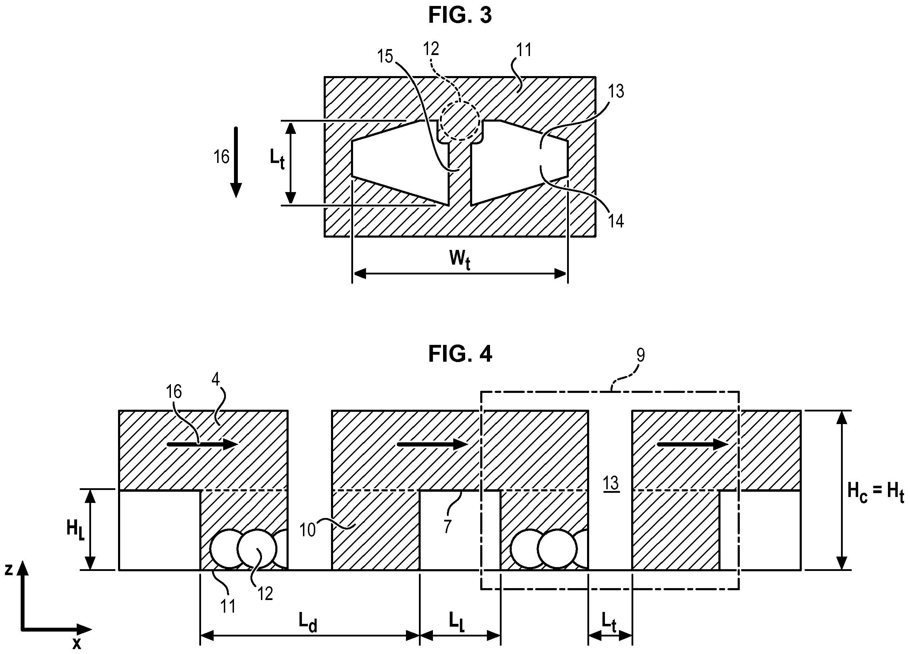

Figure 2 and figure 3 illustrate cell traps 13, according to different embodiments of the present invention, having a traversing slit 15. The width, *i.e.* the minimum dimension, of the aperture or the slit 15 is preferentially comprised between 3 µm and 30 µm, and preferentially between 5 µm and 20 µm. Therefore, the cell trap 13 can be a weir-trap since the width of the aperture can be smaller than the size of a cell 12, while the width of the aperture is large enough for streamlines to pass by the aperture.

According to another embodiment of the invention, the obstacle 14 can extend only partially from the bottom surface out of the recess 10. Therefore, streamlines can pass over the obstacle 14 so that the obstacle 14 can capture a cell and make it sediment.

The obstacle 14, preferentially the weir-trap, can have a U-shape, a cup, crescent, pierced-crescent, star, hemisphere, any polygon with three or more vertices, and/or cavity shape.

The dimensions of the obstacle 14 can be chosen in regards with the average diameter of the cultured cell D_{cell}. Such a parameter D_{cell} throughout the specification is defined as the mean cultured cell diameter, the cell 12 being in suspension for use of the method of the present invention. D_{cell} can be measured by different techniques, as impedance cell counter detection (for example, but not limited to, a Coulter counter as described in US patent 2,656,508 or by optical microscopy followed by image analysis). Depending on the source of cells, D_{cell} can typically be comprised between 3 µm and 150 µm, notably between 5 µm and 50 µm.

The width Wₜ of an obstacle 14, *i.e.* the size of an obstacle 14 in a second direction 19 transverse to the main flow direction 16 and to the first direction 8, is preferentially greater than D_{cell}, notably greater than 10 µm and preferentially greater than 20 µm.

### Recesses 10

The dimensions of the recess 10 are chosen so that a cell 12 can proliferate in the recess 10. The recess 10 is configured to receive a plurality of cells 12. According to a preferred embodiment of the invention, the area of the recess projected in the planar surface 3 is greater than 150 µm², notably greater than 250 000 µm², and preferentially greater than 500 000 µm².

Referring to figure 3, the geometry of the recesses 10 can be designed so that cells 12 in the recess 10, for example at the bottom surface 11 of the recess 10, can be protected from being transported by advection out of the recess 10 for a predefined flow rate in the fluidic channel 4. In other words, the geometry of the recess 10 allows, for a predefined flow rate, to have dead waters in the recesses 10 while a flow occurs in the upper part of the fluidic channel 4, *i.e.* outside of the recess 10. The term *"dead water"* or *"cavity region"* is used herein to designate a volume of liquid in which there exists a range of flow rates that are not great enough to drag a cell 12 out of the recess 10, and preferentially in which the flow is not great enough to drag a cell 12. Therefore, it is possible to wash, or to bring any active compound to the cells 12 in the recess 10 by injecting a washing medium and/or a medium comprising the active compound at the predefined flow rate, without dragging the cells 12 out of the recess 10. Therefore, the fabrication of the cell culture device 1 can be simplified compared to devices of the prior art, for example by avoiding the fabrication of semi-permeable membrane or walls to contain cells 12 from being dragged by a culture medium flow.

The recess extends preferentially in the second direction 19 over a distance greater than 2. D_{cell}, notably over more than 15 µm, and preferentially over more than 100 µm. According to a preferred embodiment of the invention in figure 1, each recess 10 extends over more than the half of the width W of the fluidic channel, preferentially over the entire width W of the fluidic channel. The fluidic channel can comprise lateral sides extending between the cell medium inlet(s) 5 and the cell medium outlet(s) 6. The recess 10 preferentially extends over the entire width of the fluidic channel 4, from one lateral side to the other.

Preferentially, the recess 10 is formed transverse to the main flow direction 16. Therefore, it is possible to have dead waters in the recess 10 for greater flow rate in the fluidic channel 4 than for other recess 10 geometries. Preferentially, the length L_{d} of the recess 10 is lower than the width W_{d} of the recess 10, and more preferentially the length L_{d} of the recess 10 is five times lower than the width W_{d} of the recess 10.

The fluidic channel 4 comprises an upper wall 20, relative to the first direction 8. A first height H_{c} is defined as the shorter distance between the upper surface 23 of the lower wall 7 and the upper wall 20. The recess 10 has preferentially a second height Hₜ relative to the first direction 8, defined as the shorter distance, relative to the first direction 8, between the bottom surface 11 of the lower wall 7 and the upper surface 23 of the lower wall 7. The second height Hₜ is notably comprised between 0,25 and 0,85 times the first height H_{c} and preferentially between 0,40 and 0,70 times the first height H_{c}. If the second height Hₜ of the recess 10 would be, for example, several times lower than the first height H_{c}, no dead water zone in the recess 10 could occur when injecting a liquid medium in the fluidic channel 4 to protect the cells in culture. In the other way, if the second height Hₜ of the recess 10 would be several times greater than the first height H_{c} of the fluidic channel 4, the flow of liquid medium in the fluidic channel 4 would occur outside of the recess 10, making difficult to create a vortex in the recess 10 in order to drag the cells 12 out of the recess 10. The preferred range of height ratio Hₜ/H_{c} according to an embodiment of the invention makes possible to select, depending on the flow rate of medium injected in the fluidic channel 4, if the liquid medium in the recess 10 is sensibly stable, or if vortexes can occur in the recess 10.

Preferentially, the second height Hₜ of the recess 10 is comprised between 10 µm and 1000 µm, notably between 15 µm and 300 µm and preferably between 15 µm and 200 µm.

### Array of cell traps 13

The fluidic channel 4 comprises a plurality of cell traps 13. Preferentially, the fluidic channel 4 comprises at least one array of cell traps 13, and preferably a plurality of arrays of cell traps 13. The array of cell traps 13 can be linear, for example extending relative to the second direction 19, and/or in two dimensions, for example extending in a plane parallel to the planar surface 3.

The parameters of the cell traps 13 array and/or the arrangement of the different arrays in the fluidic channel 4 define the cell capture rate at each point of the fluidic channel 4.

The minimum distance between two cell traps 13, preferentially between two obstacles 14, is a parameter of the cell capture rate during the perfusion of liquid medium comprising cells 12 in the fluidic channel 4. The minimum distance between two obstacles 14 is defined as the minimum distance between the wall of an obstacle 14 to the wall of the nearest obstacle 14. According to a preferred embodiment of the present invention, the minimum distance between two obstacles 14 is comprised between 10 µm and 300 µm, notably between 20 µm and 150 µm and preferentially between 25 µm and 100 µm. Therefore, the cell 12 capture during a perfusion can be efficient enough to seed cells 12 in the fluidic channel 4 in less than 10 minutes, preferably in less than 1 minute, while being low enough to avoid the formation of cell plugs in the entrance of the fluidic channel 4.

According to a preferred embodiment of the invention, the obstacles 14 are arranged in a regular two-dimensional lattice, in which lattice is tilted relative to the main flow direction 16. Two consecutive obstacles 14 relative to the main flow direction 16 define a tilt angle of the lattice with the main flow direction 16 different than zero, preferentially comprised between 2° and 20°, notably between 2° and 10° and more preferentially between 2° and 5°.

According to a possible embodiment of the invention, the obstacles can be arranged in the fluidic channel 4 so that the surface density of obstacles 14, projected in the planar surface 3, varies along the length of the fluidic channel. Preferentially, the surface density of the obstacles 14 is increasing relative to the main flow direction 16. Therefore, the cells 12 can be seeded at a uniform or constant concentration along the fluidic channel 4.

### Harvesting

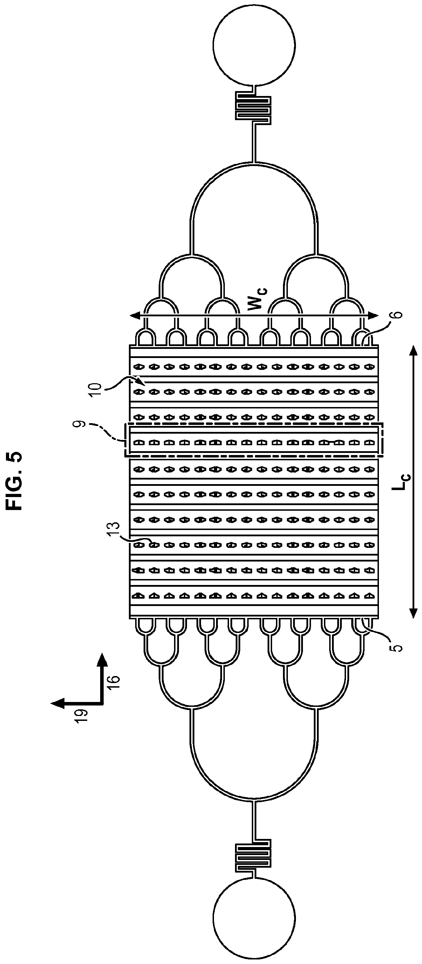

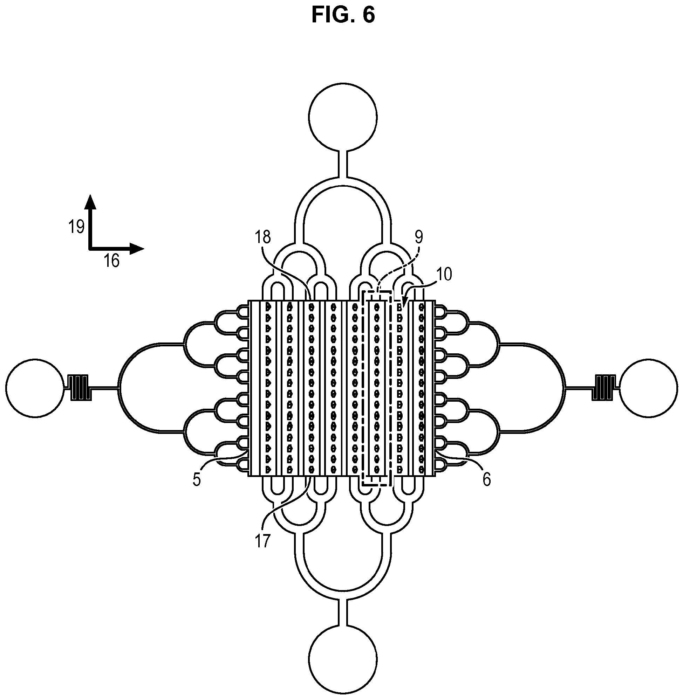

Referring to figure 5 and to figure 6, the cells 12, particularly the cells 12 cultured in the recesses 10 of the cell culture device 1, can be harvested, for example for further therapeutic use.

According to an embodiment of the invention illustrated in figure 5, the cells 12 can be harvested by flushing the recesses 10 and recouping the cells 12 from the cell medium outlet(s) 6 of the fluidic channel 4.

According to another embodiment of the invention illustrated in figure 6, the fluidic channel 4 can comprise at least a harvesting inlet 17 and at least a harvesting outlet 18, each being different from the cell medium inlet(s) 5 and from the cell medium outlet(s) 6. The harvesting inlet 17 and the harvesting outlet 18 are arranged so that a flow controlled between the harvesting inlet 17 and the harvesting outlet 18 advects the cells 12 in the recesses 10 in order to harvest them. In contrast with the flow controlled between the cell medium inlet 5 and the cell medium outlet 6, the flow controlled between the harvesting inlet 17 and the harvesting outlet 18 is configured so that no dead water occurs in the recesses 10. According to the embodiment of the invention illustrated in figure 6, the harvesting inlet 17 and the harvesting outlet 18 are configured to permit a flow relative to the second direction 19, and the recesses 10 are extending following the same direction.

In a preferred embodiment of the invention, each of the recesses 10 is fluidically connected to a harvesting inlet 17 and to a harvesting outlet 18. Therefore, dead waters are avoided in the recesses 10 when controlling a flow between the harvesting inlet 17 and the harvesting outlet 18. The recesses 10 can be fluidically connected to the same common harvesting inlet 17 and common harvesting outlet 18, and/or being connected individually by different harvesting inlets 17 and harvesting outlets 18.

### Parallelization

The cell culture is adapted for cell therapy, *i.e.* the cell culture device 1 is adapted to harvest more than 10⁵ cells 12, notably more than 10⁶ cells 12, and preferably more than 10¹⁰ cells 12. The fluidic channel 4 can be parallelized to increase the throughput of the cell culture device 1. According to a preferred embodiment of the invention, the cell culture device 1 comprises an inlet channel 21 and an outlet channel 22. The inlet channel 21 connects fluidically the cell medium inlets 5 and the outlet channel 22 connects fluidically the cell medium outlets 6. Therefore, the production of cell 12 can be parallelized. The different fluidic channels 4 can be stacked for example. Different fluidic channels 4 can also be arranged in the same plane of the same stack.

### Surface treatment

Different types of cells 12 can be cultured in the cell culture device 1, for example adherent cells 12 or non-adherent cells 12. The bottom surface 11 of the recesses 10, notably the recesses 10, and preferentially the surfaces of the walls of the fluidic channel 4, can be coated with a surface treatment adapted to the type of cells 12 cultured in the cell culture device 1. Typically, the surface treatment can comprise a step of injection and circulation of a liquid medium adapted for the surface treatment through the culture device. The liquid medium can be introduced in the fluidic channel 4 either through the cell medium inlet 5 and the cell medium outlet 6 or through the harvesting inlet 17 and the harvesting outlet 18, with the surface treatment being passively adsorbed onto the culture surface, i.e. on the wall(s) of the recess 10 and specifically on the bottom surface 11. This is especially used for extracellular matrix coating. Other steps can comprise of plasma polymerization and chemically-induced adsorption such as, but not limited to, polymer grafting.

### Cell culture

The above-mentioned cell culture device 1 is adapted to culture the cells 12, for example for use in cell therapy.

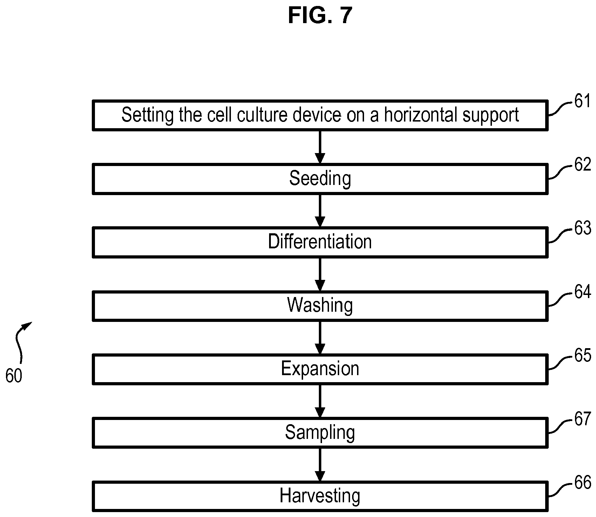

Figure 7 illustrates a method of cell culture according to an embodiment of the invention. The method can comprise a step 61 of setting the cell culture device 1 so that the surface portion 2 lies on a horizontal support. Therefore, the recesses 10 are arranged under the rest of the fluidic channel 4 relative to the first direction 8, and the cell trap 13 is adapted to make the cell 12 sediment to the bottom surface 11 of the recess. As an alternative, the surface portion 2 can be tilted and the fluidic channel 4 is also tilted so that the recesses 10 are arranged under the rest of the fluidic channel 4 relative to the first direction 8.

The method of cell culture comprises a seeding step 62. At least a cell 12 can be brought to a recess 10 by the mean of the cell trap 13, notably to the majority of the recesses 10 and preferentially to more than 80 % of the recesses 10. A liquid medium comprising cells 12 is injected in the cell medium inlet(s), until at least until more than 10% of the cell traps 13 have captured at least one cell 12, notably more than one third of the cell traps 13 have captured at least one cell 12, and preferentially until the majority of the cell traps 13 have captured at least one cell 12. Therefore, the cells 12 can be seeded in the cell culture device 1 by injecting the medium comprising the cells 12 at a velocity independent from the effect of the gravity forces on the cells 12. The cell culture device 1 can therefore be seeded efficiently and homogeneously. The number of seeded cells 12 and/or the number of cells 12 captured by the cell traps 13 can be measured during the injection by conventional microscopy methods, for example by recording images of the fluidic channel 4 using an inverted microscope. The captured cells 12 can then be counted by automatic image treatment and/or manually. The injection time can be predefined.

After the seeding step 62, the method can comprise an expansion step 65 or proliferation step 65. An appropriate culture environment is critical for the cells 12 expansion: the expansion step 65 comprises the injection of a medium comprising at least one active agent in the cell medium inlet. The terms *"active agent"* are used herein for any chemical or biological compound which is compulsory or beneficial for the expansion of the cells 12. The injection of the medium is controlled at a flow rate low enough to avoid dragging cells 12 out of the recesses 10, and preferentially to not induce vortex in the recesses 10. The liquid flow essentially occurs in the rest of the fluidic channel 4. Therefore, the active agents can be transported to the cells 12 by diffusion from the rest of the fluidic channel 4 to the recesses 10, without moving or advecting the cells 12 in expansion in the recesses 10.

After the seeding step 62, the method can further comprise a washing step 64. In order to precisely control a chemical or biological treatment to the cells 12, the washing of the fluidic channel 4 can be necessary. The washing step 64 comprises the injection of a washing medium, said washing medium being free from the compound to be washed. The injection of the washing medium is controlled at a flow rate low enough to avoid dragging cells 12 out of the recesses 10, and preferentially to not induce vortex in the recesses 10. The liquid flow essentially occurs in the rest of the fluidic channel 4. Therefore, the compound to be washed by dilution or can be transported by diffusion from the recesses 10 to the rest of the fluidic channel 4, without moving or advecting the cells 12 in the recesses 10.

After the seeding step 62, the method can further comprise a differentiation step 63. The term *"differentiation"* is used herein as the process where a cell changes from a less specialized cell type to a more specialized cell type. The differentiation step 63 comprises the injection of a liquid medium comprising a differentiation agent. The injection of the medium comprising a differentiation agent is controlled at a flow rate low enough to avoid dragging cells 12 out of the recesses 10, and preferentially to not induce vortex in the recesses 10. The liquid flow essentially occurs in the rest of the fluidic channel 4. Therefore, the differentiation agent can be transported to the cells 12 by diffusion from the rest of the fluidic channel 4 to the recess 10, without moving or advecting the cells 12 in the recesses 10.

The method can further comprise a harvesting step 66. The harvesting step 66 can comprise the injection of a medium in the cell medium inlet(s) 5 and recouping cells from the cell medium outlet. Harvesting of the cells 2 can be handled by injecting a medium at a flow rate high enough to create vortex in the recesses 10 and to drag more than the majority of the cells 12 out of the recesses 10 to the rest of the fluidic channel 4. The cells 12 can be then advected by the flow of liquid medium and transported to the cell medium outlet 6 where they can be recouped.

A threshold flow rate between the cell medium inlet 5 and the cell medium outlet 6 separates:
- an operation mode in which the liquid flow in the recess 10 is sensibly locally zero (dead water), or in which the drag forces applied on the cells 12 in the recess 10 are not sufficient to lift the cells 12 out of the recess 10, and
- another operation mode in which the liquid flow in the recess 10 is related to a vortex, and the drag forces of the vortex on the cells 12 are high enough to lift the cells out of the recess 10.

The threshold flow rate can be determined considering the geometry of the fluidic channel 4 and the type of cultured cells 12, for example analytically or by using computer simulation using finite element methods. One appropriate method can be for example to determine at which flow rate between the cell medium inlet 5 and the cell medium outlet 6 the local drag forces on a cell 12 relative to the first direction 8 can compensate the gravity forces on the same cell 12. The flow rate at which the medium is injected in the harvesting step 66, *i.e.* the second flow rate, is greater than the first predefined flow rate of the expansion step 65 and/or of the washing step 64 and/or of the differentiation step 63.

Alternatively, the harvesting step 66 can comprise the injection of a medium in the harvesting inlet(s) 17, and recouping cells 12 from the harvesting outlet(s) 18.

The method can also comprise a sampling step 67 During the cell culture, the sampling of the cultured cells 12 can be compulsory to determine, for example, the activity, potency, viability, selectivity, morphology and/or more generally the different characteristics of the cells 12. The sampling step 67 can comprise the injection of a medium in the fluidic channel 4 at a third flow rate, greater than the first flow rate. The third flow rate can be chosen for example between the threshold flow rate and 1,5 times the threshold flow rate. By adjusting the perfusion time during the sampling step 67, less than one third of the cells 12 in the recesses 10 are recouped and preferentially less than 10% of the cell 12 are recouped. The sampling step 67 can also comprise the sampling of the liquid medium in the microchannel 4, in order to further analyze the sterility and composition of the cell culture fluid and environment.

## Claims

1. A cell culture device (1), having at least one surface portion (2) defining a planar surface (3), the surface portion being configured for lying on a horizontal support, comprising at least a fluidic channel (4) having at least a cell medium inlet (5) and a cell medium outlet (6), said fluidic channel comprising a lower wall (7) extending between the cell medium inlet and the cell medium outlet,
the cell culture device being **characterized in that** it further comprises:
- at least one recess (10) configured to receive a plurality of cells (12), said recess (10) being formed by the lower wall (7) and defining a bottom surface (11), and
- at least a cell trap (13), said cell trap (13) being configured to capture a cell (12) advected in the fluidic channel and then to make the cell sediment to the bottom surface (11) of the recess (10).

2. The cell culture device according to claim 1, wherein the lower wall (7) further comprises an upper surface (23) extending aside of the recess (10) and the cell trap (13) comprises an obstacle (14) extending from the bottom surface (11) and out of the recess (10) beyond the upper surface of the lower wall (7).

3. The cell culture device according to claims 1 or 2, wherein the fluidic channel further comprises an upper wall (20), and the cell trap (13) comprises an obstacle (14) extending at least from the upper wall (20) to the recess (10).

4. The cell culture device according to any of claims 1 to 3, wherein the obstacle (14) has a main direction of extension defining a sedimentation axis, which is parallel to a sedimentation direction of the cell within said obstacle, and wherein said sedimentation axis is perpendicular to the surface portion (2).

5. The cell culture device according to any of claims 1 to 4, wherein each obstacle has an aperture, notably a traversing aperture and preferentially a traversing slit (15), a width of the aperture being comprised between 3 µm and 30 µm, and preferentially between 5 µm and 20 µm.

6. The cell culture device according to any of claims 1 to 5, wherein at least a portion of the fluidic channel (4) is configured for receiving a liquid flow according to a main flow direction (16) extending between the cell medium inlet (5) and the cell medium outlet (6), and
wherein the fluidic channel (4) comprises an array of cell traps (13), said array of cell traps (13) extending along a direction which is perpendicular to the main flow direction (16) in a plane parallel to the planar surface (3).

7. The cell culture device according to any of claims 1 to 6, wherein the fluidic channel further comprises lateral sides extending between the cell medium inlet(s) (5) and the cell medium outlet(s) (6), and wherein at least a recess extends over the entire width of the fluidic channel from one lateral side to the other.

8. The cell culture device according to any of claims 1 to 7, wherein the lower wall (7) further comprises an upper surface extending aside of the recess (10) and wherein the fluidic channel further comprising an upper wall (20), wherein a first height of the fluidic channel (4) is defined as a shorter distance between the upper surface (23) of the lower wall (7) and the upper wall (20), a second height of recess (10) is defined as a shorter distance between the bottom surface (11) of the lower wall and the upper surface (23) of the lower wall, and wherein the second height is comprised between 0.15 and 0.85 times the first height and preferentially between 0.20 and 0.70 times the first height.

9. The cell culture device according to any of claims 1 to 8, wherein the lower wall (7) further comprises an upper surface extending aside of the recess (10), wherein a second height of the fluidic channel is defined as a shorter distance between the lower surface of the lower wall and the upper surface of the lower wall, and wherein the second height is comprised between 10 µm and 500 µm, notably between 15 µm and 300 µm and preferably between 15 µm and 200 µm.

10. The cell culture device according to any of claims 1 to 9, comprising at least a harvesting inlet (17) and a harvesting outlet (18), the harvesting inlet and the harvesting outlet being different from the cell medium inlet and the cell medium outlet, the at least one recess being fluidically directly connected to a harvesting inlet (17) and to a harvesting outlet (18).

11. A method of cell culture, comprising:
- setting a cell culture device according to any of claims 1 to 10 so that the surface portion (2) lies on a horizontal support,
- a seeding step a) of injecting a medium comprising cells, said medium being injected in the cell medium inlet of the at least one fluidic channel of the cell culture device, until at least more than 10% of the cell traps have captured at least one cell, and preferentially more than the majority of the cell traps have captured at least one cell.

12. The method of cell culture according to claim 11, further comprising, further to seeding step a), an expansion step b) of injecting a medium comprising an active culture agent in the cell medium inlet, wherein the medium is injected at a first predefined flow rate, the method comprising an optional harvesting step e), further to seeding step a), of injecting a medium in the cell medium inlet at a predefined second flow rate and recouping cells from the cell medium outlet, the second flow rate being strictly higher than the first flow rate, the method further comprising an optional sampling step f), further to step a), of injecting a medium in the cell medium inlet at a predefined fifth flow rate and recouping a sample of cells from the cell medium outlet, the fifth flow rate being strictly higher than the first flow rate, the injection time and the fifth sampling flow rate being calculated previously to seeding step a) so that less than one third of the cells in the recesses are recouped.

13. The method of cell culture according to claim 11 or 12, further comprising further to seeding step a), a washing step c) of injecting a washing medium in the cell medium inlet, wherein the medium is optionally injected at a third predefined flow rate, the method further comprising an optional harvesting step e), further to seeding step a), of injecting a medium in the cell medium inlet at a predefined second flow rate and recouping cells from the cell medium outlet, the second flow rate being strictly higher than the third flow rate, the method further comprising an optional sampling step f), further to step a), of injecting a medium in the cell medium inlet at a predefined fifth flow rate and recouping a sample of cells from the cell medium outlet, the fifth flow rate being strictly higher than the third flow rate, the injection time and the fifth sampling flow rate being calculated previously to seeding step a) so that less than one third of the cells in the recesses are recouped.

14. The method of cell culture according to any of claims 11 to 13, further comprising, further to seeding step a), a differentiation step d) of injecting a medium comprising at least a differentiation factor in the cell medium inlet, wherein the medium is optionally injected at a fourth predefined flow rate, the method further comprising a harvesting step e), further to seeding step a), of injecting a medium in the cell medium inlet at a predefined second flow rate and recouping cells from the cell medium outlet, the second flow rate being strictly higher than the fourth flow rate, the method further comprising an optional sampling step f), further to step a), of injecting a medium in the cell medium inlet at a predefined fifth flow rate and recouping a sample of cells from the cell medium outlet, the fifth flow rate being strictly higher than the fourth flow rate, the injection time and the fifth sampling flow rate being calculated previously to seeding step a) so that less than one third of the cells in the recesses are recouped.

15. The method of cell culture according to any of claims 11 to 14, further comprising, further to seeding step a), a harvesting step e) of injecting a medium in the cell medium inlet(s) and recouping cells from the cell medium outlet(s).
